# EUROPÄISCHE PATENTSCHRIFT

(11) **EP 2 855 409 B1**
(45) Veröffentlichungstag und Bekanntmachung des Hinweises auf die Patenterteilung: **13.07.2016**
(21) Anmeldenummer: 13724287.1
(22) Anmeldetag: 23.05.2013
(51) Int. Cl.: C07C 29/34, C07C 29/86, C07C 31/125

(54) **VERFAHREN ZUR AUFARBEITUNG VON GEMISCHEN**
METHOD FOR PROCESSING MIXTURES
PROCÉDÉ DE PRÉPARATION DE MÉLANGES

(30) Priorität: 30.05.2012 EP 12169997
(43) Veröffentlichungstag der Anmeldung: 08.04.2015
(73) Patentinhaber: BASF SE, 67056 Ludwigshafen am Rhein (DE)
(72) Erfinder: SCHAUB, Thomas, 67433 Neustadt (DE); DIMITROVA, Pepa, 67550 Worms (DE); PACIELLO, Rocco, 67098 Bad Dürkheim (DE); BAUER, Frederic, 67146 Deidesheim (DE)
(74) Vertreter: BASF IP Association
(86) Internationale Anmeldenummer: PCT/EP2013/060651
(87) Internationale Veröffentlichungsnummer: WO 2013/178531

(56) Entgegenhaltungen:
- EP-A1- 2 221 289
- US-A- 3 479 412
- US-A- 3 514 493
- US-A- 3 862 994

## Beschreibung

Die vorliegende Erfindung betrifft ein Verfahren zur Herstellung eines verzweigten Alkohols der allgemeinen Formel (II)

R¹-CH₂-CH₂-CHR¹-CH₂-OH (II)

aus mindestens einem Alkohol der Formel (I)

R¹-CH₂-CH₂-OH (I),

weiterhin mindestens einer Komplexverbindung von mindestens einem Metall der 8., 9. oder 10. Gruppe des Periodensystems der Elemente, die gewählt ist aus Komplexverbindungen, die mindestens einen Liganden L¹ haben, der mindestens zweizähnig ist, wobei mindestens eine Koordinationsstelle von L¹ ein Stickstoffatom ist, und wobei die Komplexverbindung in mindestens einem der Lösungsmittel Toluol, m-Xylol, n-Hexan oder Alkohol der allgemeinen Formel (I) oder verzweigtem Alkohol der allgemeinen Formel (II) eine Löslichkeit von mindestens 0,5 g/l aufweist, gemessen bei Zimmertemperatur,
wobei man das durch die Reaktion erhältliche Gemisch, das mindestens den Alkohol der allgemeinen Formel (I) oder mindestens den verzweigten Alkohol der allgemeinen Formel (II) und weiterhin die Komplexverbindung und
mindestens eine Säure der allgemeinen Formel (III)

R¹-CH₂-COOH (III)

in Form eines ihrer Salze enthält,
wobei die Gruppen R¹ verschieden oder vorzugsweise gleich sind und gewählt aus C₂-C₁₀-Alkyl, linear oder verzweigt,
aufarbeitet, indem man
(a) das Gemisch mit Wasser behandelt, das ein Alkalimetallhydroxid enthalten kann,
(b) das oder die Salze von Säure der allgemeinen Formel (III) extrahiert,
und dass man das so erhältliche Gemisch einsetzt, um durch Zugabe von Alkohol der allgemeinen Formel (I) und gegebenenfalls von Base die Guerbet-Reaktion zur Herstellung von verzweigtem Alkohol der allgemeinen Formel (II) wieder zu starten.

Verzweigte Fettalkohole finden vielseitige Verwendungen als Zwischenprodukte, beispielsweise zur Herstellung von Tensiden. Es ist daher von Interesse, wirtschaftliche Verfahren zur Herstellung von verzweigten Fettalkoholen und insbesondere von in 2-Position verzweigten Fettalkoholen zu entwickeln. Von besonderem Interesse sind dabei Verfahren zur Herstellung von Guerbet-Alkoholen, die neben der Verzweigung in 2-Position weitere Verzweigungen aufweisen.

Aus J. Org. Chem. 2006, 71, 8306 ist bekannt, dass man mit Hilfe von Iridium-Komplexen Guerbet-Reaktionen katalysieren kann. Speziell ist aus der zitierten Stelle bekannt, dass man mit Hilfe von [Cp*IrCl₂]₂ und 1,7-Oktadien und Kalium-tert.-Butanolat in p-Xylol als Lösungsmittel den verzweigten Alkohol Isoamylalkohol zum entsprechenden Guerbetalkohol dimerisieren kann (Cp*: Pentamethylcyclopentadienyl). Die Ausbeute ist jedoch nicht optimal.

In US 3,514,493 wird die Herstellung von 2-Ethylhexanol und von 2-Butyloktanol mit Hilfe von geträgerten Metallen, beispielsweise mit Hilfe von auf Aktivkohle geträgertem Palladium oder Ruthenium, offenbart. In J. Organomet. Chem. 1972, 37, 385 wird vorgeschlagen, dass man mit Hilfe von RuCl₃ mit bestimmten Phosphan-Liganden in homogener Phase Guerbet-Alkohole machen kann. In EP 2 221 289 A1 wird ein Verfahren zur Herstellung von Guerbetalkoholen mit Hilfe von Ruthenium- oder Rhodiumkomplexen beschrieben.

Die Aufarbeitung des Reaktionsgemisches erfolgt in vielen Fällen destillativ. Zunächst destilliert man nicht verbrauchtes Ausgangsmaterial ab, dann den gewünschten Guerbet-Alkohol. Der Katalysator bleibt in den meisten Fällen unbeschädigt, so dass es möglich ist, Alkohol und frische Base zuzugeben und die Guerbet-Reaktion erneut ablaufen zu lassen. Führt man die Guerbet-Reaktion in mehreren Zyklen durch - also beispielsweise Durchführung der eigentlichen Reaktion, destillative Aufarbeitung, Zugabe von Alkohol als Ausgangsmaterial, Durchführung der eigentlichen Reaktion, destillative Aufarbeitung usw. - beobachtet man häufig, dass Umsatz und Ausbeute pro Zyklus gemindert werden. Dadurch lässt die Effizienz des Katalysators nach, was angesichts des hohen Preises vieler Katalysatoren (Zentralmetall, Ligand) nachteilig ist. Man kann weiterhin beobachten, dass nach mehreren Zyklen ein immer weiter steigender Anteil von Feststoffen im Reaktionsgefäß verbleibt, der die Durchmischung der Reaktionsmischung erschwert.

Es bestand also die Aufgabe, ein Verfahren bereit zu stellen, durch das die vorstehend diskutierten Nachteile vermieden werden können. Ein derartiges Verfahren sollte einfach durchzuführen sein und ein Katalysator-haltiges Gemisch liefern, welches rasch wieder zur Synthese von Guerbet-Alkoholen einsetzbar ist.

Dementsprechend wurde das eingangs definierte Verfahren gefunden, kurz auch erfindungsgemäßes Verfahren genannt.

Das erfindungsgemäße Verfahren geht aus von mindestens einem Alkohol der allgemeinen Formel (I), der unten beschrieben ist. Erfindungsgemäß setzt man ihn unter Katalyse durch mindestens eine öllösliche Komplexverbindung von mindestens einem Metall der 8., 9. oder 10. Gruppe des Periodensystems der Elemente um. Der Begriff "öllösliche Komplexverbindung" von mindestens einem Metall der 8., 9. bzw. 10. Gruppe des Periodensystems der Elemente wird unten definiert.

Durch die Reaktion erhält man ein Gemisch, welches mehrere Bestandteile enthält. Ein Bestandteil ist mindestens ein Alkohol der allgemeinen Formel (I)

R¹-CH₂-CH₂-OH (I)

in der R¹ gewählt wird aus C₂-C₁₀-Alkyl, linear oder - soweit möglich - verzweigt, also Ethyl, n-Propyl, Isopropyl, n-Butyl, iso-Butyl, sec.-Butyl, n-Pentyl, n-Hexyl, n-Heptyl, n-Octyl, n-Decyl, bevorzugt sind Ethyl und Isopropyl, ganz besonders bevorzugt Isopropyl.

In einer bevorzugten Ausführungsform der vorliegenden Erfindung ist R¹ gewählt aus Ethyl, n-Propyl, n-Butyl und Isopropyl.

Alkohol der allgemeinen Formel (I) kann in reiner Form oder in Form von Gemischen vorliegen, insbesondere in Form von Isomerengemischen, insbesondere in Form von Gemischen mit mindestens einem isomeren Alkohol. Dabei können im Falle von R¹ = Propyl der oder die isomeren Alkohole der Formel (I) entsprechen. In einer besonderen Variante der vorliegenden Erfindung liegt Alkohol der allgemeinen Formel (I) im Gemisch mit mindestens einem solchen isomeren Alkohol vor, der nicht der Formel (I) entspricht.

Beispiel für einen geeigneten isomeren Alkohol von Isoamylalkohol (R¹ = Isopropyl) ist 2-Methylbutanol. Dieser reagiert mit Isoamylalkohol vorzugsweise zu einem Alkohol der Formel (IV.1a)

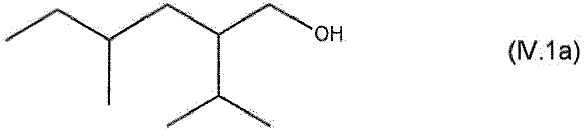

In einer Ausführungsform der vorliegenden Erfindung liegt Alkohol der Formel (I) in einer Mischung mit 0,1 bis 25 mol-% von mindestens einem isomeren Alkohol vor, der der Formel (I) entsprechen kann, aber vorzugsweise nicht entspricht.

Alkohol der allgemeinen Formel (I) und insbesondere Mischungen von Alkohol der allgemeinen Formel (I) mit einem oder mehreren seiner Isomeren kann man durch Synthese oder aufgrund von biologischen Rohstoffen herstellen, beispielsweise durch Fermentation oder anderen biologischen Abbau von Sacchariden.

Erfindungsgemäß eingesetztes Gemisch enthält weiterhin mindestens eine öllösliche Komplexverbindung von mindestens einem Metall der 8., 9. oder 10. Gruppe des Periodensystems der Elemente, kurz auch "Komplex" genannt, die gewählt ist aus Komplexverbindungen, die mindestens einen Liganden L¹ haben, der mindestens zweizähnig ist, wobei mindestens eine Koordinationsstelle von L¹ ein Stickstoffatom ist.

Dabei wird unter "ällöslich" verstanden, dass die Löslichkeit vom Komplex in mindestens einem der Lösungsmittel Toluol, m-Xylol, n-Hexan oder Alkohol der allgemeinen Formel (I) oder verzweigtem Alkohol der allgemeinen Formel (II) mindestens 0,5 g/l beträgt, gemessen bei Zimmertemperatur. Mindestens einer der Komplexe dient als Katalysator. Dabei ist es möglich, dass in der Reaktionsmischung zwei oder mehr öllösliche Komplexverbindungen von Metall der 8., 9. oder 10. Gruppe des Periodensystems der Elemente vorliegen, von denen aber nur eine katalytisch aktiv ist.

In einer Ausführungsform der vorliegenden Erfindung hat Komplex bei 25°C einen Nernstschen Verteilungskoeffizienten von mindestens 3, bevorzugt mindestens 5, besonders bevorzugt mindestens 10, ganz besonders bevorzugt mindestens 20, ganz besonders bevorzugt bis 1.000.000, bezogen auf mindestens eines der Lösungsmittel Toluol, m-Xylol, n-Hexan oder Alkohol der allgemeinen Formel (I) oder verzweigtem Alkohol der allgemeinen Formel (II) einerseits und Wasser andererseits.

Komplex weist mindestens ein Metall der 8., 9. oder 10. Gruppe des Periodensystems auf und mindestens einen Liganden L¹. Metalle der 8., 9. oder 10. Gruppe sind gewählt aus Eisen, Kobalt, Nickel, Ruthenium, Rhodium, Iridium, Osmium, Palladium und Platin, bevorzugt ist Iridium und besonders bevorzugt ist Ruthenium. Iridium liegt vorzugsweise als Iridium(I) oder Iridium(III) vor, Ruthenium bevorzugt als Ru(0), Ru(III) und besonders bevorzugt als Ru(II).

In einer Ausführungsform der vorliegenden Erfindung weist Komplex mindestens einen Liganden L¹ auf. Dabei ist Ligand L¹ über zwei, drei oder vier Stickstoffatome mit dem Zentralmetall, also mit Metall der 8., 9. oder 10. Gruppe des Periodensystems der Elemente, bevorzugt mit Iridium oder Ruthenium und insbesondere mit Ru(II) koordiniert, bevorzugt über zwei oder drei, und L¹ weist keine von Stickstoff verschiedenen Koordinationsstellen auf. Beispiel für einen zweizähnigen Liganden L¹, der über zwei Stickstoffatome mit Ru(II) koordiniert und keine von Stickstoff verschiedenen Koordinationsstellen aufweist, ist 2,2'-Bipyridyl.

In einer anderen Ausführungsform der vorliegenden Erfindung ist Ligand L¹ über zwei oder drei Koordinationsstellen mit dem Zentralmetall, also mit Metall der 8., 9. oder 10. Gruppe des Periodensystems der Elemente, bevorzugt mit Iridium oder Ruthenium und insbesondere mit Ru(II) koordiniert, von denen eine oder zwei Koordinationsstelle(n) von Stickstoff verschieden ist/sind und die übrige(n) sind/ist Stickstoffatom(e). Von Stickstoff verschiedene Koordinationsstellen von Ligand L¹ sind gewählt aus Phosphor-Atomen, Sauerstoffatomen, Schwefel-Atomen und insbesondere Carben-Kohlenstoff-Atomen.

Stickstoffatome, die an Zentralmetall koordinieren, sind dabei vorzugsweise gewählt aus tertiären Amin-Stickstoff-Atomen, die Teil eines Heterocyclus' sind, und Stickstoffatomen, die Teil einer tertiären Aminogruppe sind, die nicht Teil eines Heterocyclus' ist.

In einer Ausführungsform der vorliegenden Erfindung wählt man L¹ aus Verbindungen der allgemeinen Formel (V) 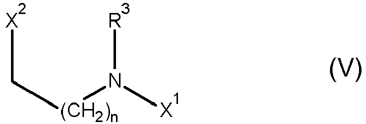 wobei die Variablen wie folgt gewählt sind:
- R³: gewählt aus
Wasserstoff,
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt n-C₁-C₄-Alkyl wie Methyl, Ethyl, n-Propyl, n-Butyl, insbesondere Methyl;
C₃-C₁₀-Cycloalkyl, beispielsweise Cyclopropyl, Cyclobutyl, Cyclopentyl, Cyclohexyl, Cyc-loheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
- n: gewählt aus null und eins,
- X¹: gewählt aus
Wasserstoff,
C₁-C₅-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, insbesondere Methyl oder Isopropyl, und
(CH₂)ₙ₊₁-X²,
- X²: gewählt aus NR⁴R⁵, 2-Pyridyl und Imidazol-2-ylidenyl der Formel

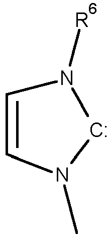

R⁴, R⁵ verschieden oder vorzugsweise gleich und gewählt aus
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt tert.-Butyl oder n-C₁-C₄-Alkyl wie Methyl, Ethyl, n-Propyl, n-Butyl, insbesondere tert.-Butyl oder Methyl;
C₃-C₁₀-Cycloalkyl, beispielsweise Cyclopropyl, Cyclobutyl, Cyclopentyl, Cyclohexyl, Cycloheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl,
R⁶ gewählt aus C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt n-C₁-C₄-Alkyl wie Methyl, Ethyl, n-Propyl, n-Butyl, insbesondere Methyl;
C₃-C₁₀-Cycloalkyl, beispielsweise Cyclopropyl, Cyclobutyl, Cyclopentyl, Cyclohexyl, Cycloheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl.

In einer Ausführungsform der vorliegenden Erfindung wählt man L¹ aus Verbindungen der allgemeinen Formel (VI) 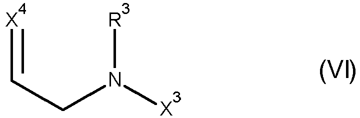 wobei die Variablen wie folgt gewählt sind:
- R³: gewählt aus
Wasserstoff,
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt n-C₁-C₄-Alkyl wie Methyl, Ethyl, n-Propyl, n-Butyl, insbesondere Methyl
C₃-C₁₀-Cycloalkyl, beispielsweise Cyclopropyl, Cyclobutyl, Cyclopentyl, Cyclohexyl, Cyc-loheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl,
- X³: gewählt aus
Wasserstoff,
C₁-C₅-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl; bevorzugt n-C₁-C₄-Alkyl wie Methyl, Ethyl, n-Propyl, n-Butyl, insbesondere Methyl, und
CH₂=X⁴,
- X⁴: gewählt aus NR⁴, und
- R⁴: gewählt aus
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt tert.-Butyl oder n-C₁-C₄-Alkyl wie Methyl, Ethyl, n-Propyl, n-Butyl, insbesondere Methyl oder tert.-Butyl,
C₃-C₁₀-Cycloalkyl, beispielsweise Cyclopropyl, Cyclobutyl, Cyclopentyl, Cyclohexyl, Cycloheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl.

In einer Ausführungsform der vorliegenden Erfindung wählt man L¹ aus Verbindungen der allgemeinen Formel (VII) 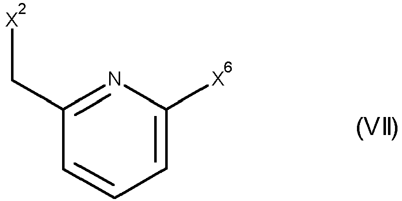 wobei die Variablen wie folgt gewählt sind:
- X⁶: gewählt aus Wasserstoff,
C₁-C₅-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl; bevorzugt n-C₁-C₄-Alkyl wie Methyl, Ethyl, n-Propyl, n-Butyl, insbesondere Methyl, und
CH₂-X²,
- X²: gewählt aus NR⁴R⁵, 2-Pyridyl und Imidazol-2-ylidenyl der Formel

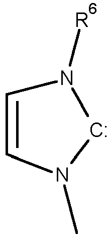

R⁴, R⁵ verschieden oder vorzugsweise gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.
R³, R⁴, R⁵ und R⁶ sind wie vorstehend näher definiert.

In einer Ausführungsform der vorliegenden Erfindung wählt man L¹ aus Verbindungen der allgemeinen Formel (VIII) 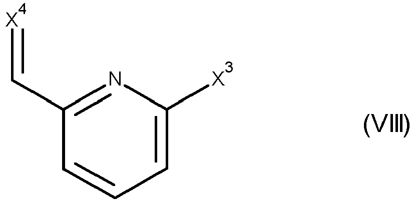 wobei die Variablen wie folgt gewählt sind:
- X³: gewählt aus Wasserstoff, C₁-C₅-Alkyl und CH₂=X⁴,
- X⁴: gleich oder gegebenenfalls verschieden, vorzugsweise gleich, und gewählt aus N-R⁴,
- R⁴: C₁-C₁₀-Alkyl, C₃-C₈-Cycloalkyl, Benzyl oder Phenyl, unsubstituiert oder ein- oder mehrfach substituiert mit C₁-C₃-Alkyl.

In einer Ausführungsform der vorliegenden Erfindung wählt man L¹ aus Verbindungen der allgemeinen Formel (IX) 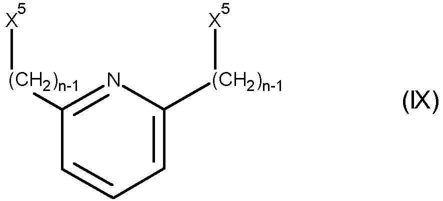 wobei die Variablen wie folgt gewählt sind:
- n: verschieden oder vorzugsweise gleich und jeweils null oder eins
- X⁵: jeweils gleich und gewählt aus NR⁴R⁵, 2-Pyridyl und Imidazol-2-ylidenyl der Formel

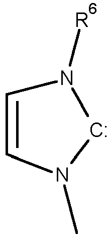

R⁴, R⁵ verschieden oder gleich und gewählt aus Wasserstoff, C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.
R³, R⁴, R⁵ und R⁶ sind wie vorstehend näher definiert.

Besonders bevorzugte Beispiele für Liganden L¹ sind solche der Formel (IX.1) 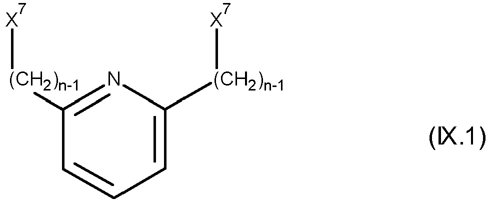
bei denen die Gruppen (CH₂)ₙ₋₁-X⁷ jeweils gleich sind und gewählt aus
2-Pyridyl (also jeweils n = null),
CH₂-N(CH₃)₂, CH₂-N(C₂H₅)₂, CH₂-N(n-C₃H₇)₂, CH₂-N(n-C₄H₉)₂, CH₂-N(iso-C₃H₇)₂, CH₂-N(tert.-C₄H₉)₂, CH₂-N(n-C₅H₁₁)₂, CH₂-N(n-C₆H₁₃)₂, CH₂-N(n-C₈H₁₇)₂, CH₂-N(C₆H₅)₂, CH₂-N(CH₂-C₆H₅)₂, und CH₂-N(cyclo-C₆H₁₁)₂, (also jeweils n = 1), und 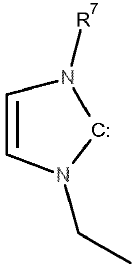 (also jeweils n = 1), mit R⁷ gewählt aus
Methyl, Ethyl, n-Propyl, iso-Propyl, n-Butyl, n-Pentyl, n-Hexyl, n-Heptyl, n-Octyl, bevorzugt Iso-propyl oder n-C₁-C₄-Alkyl wie Methyl, Ethyl, n-Propyl, n-Butyl, insbesondere Methyl,
Cyclohexyl und
Phenyl, unsubstituiert oder ein- oder bis zu dreifach substituiert mit gleichem oder verschiedenem C₁-C₃-Alkyl, beispielsweise para-Methylphenyl, 2,6-Dimethylphenyl, 2,4,6-Trimethylphenyl, 2,6-Diethylphenyl, 2,6-Diisopropylphenyl und 2-Methyl-4-isopropylphenyl.

Andere besonders bevorzugte Beispiele für Liganden L¹ sind solche der Formel (VIII.1)

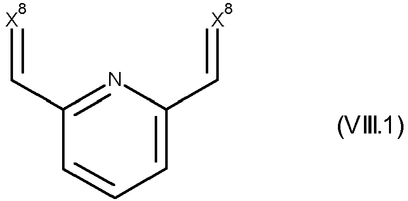

in denen X⁸ jeweils gleich sind und gewählt aus N-CH₃, N-C₂H₅, N-n-C₃H₇, N-n-C₄H₉, N-iso-C₃H₇, N-n-C₅H₁₁, N-n-C₆H₁₃, N-n-C₈H₁₇, N-CH₂-C₆H₅ und N-cyclo-C₆H₁₁, und
N-Phenyl, unsubstituiert oder ein- oder bis zu dreifach substituiert mit gleichem oder verschiedenem C₁-C₃-Alkyl, beispielsweise N-para-Methylphenyl, N-2,6-Dimethylphenyl, N-2,4,6-Trimethylphenyl, N-2,6-Diethylphenyl, N-2,6-Diisopropylphenyl und N(2-methyl-4-isopropylphenyl).

Ein ganz besonders bevorzugter Ligand L¹ ist 2,6-bis-2-Pyridylpyridin, im Rahmen der vorliegenden Erfindung auch kurz "Terpyridyl" genannt.

In einer Ausführungsform der vorliegenden Erfindung kann Komplex mindestens einen weiteren Liganden aufweisen, gewählt aus CO, Pseudohalogeniden, organischen Carbonylverbindungen, Aromaten, Olefinen, Phosphanen, Hydrid und Halogeniden.

Unter "mindestens einem weiteren Liganden" ist dabei ein Ligand zu verstehen, der von Ligand L¹ verschieden ist. Beispiele für weitere Liganden sind
- CO (Kohlenmonoxid),
- Pseudohalogenid, insbesondere Cyanid, Isocyanat und Rhodanid,
- organische Carbonylverbindungen, beispielsweise Ketone, bevorzugt organische Dicarbonylverbindungen wie Acetylacetonat, 1-Phenylbutan-1,3-dion, Acetessigester,
- Aromaten, die elektrisch geladen sein können oder ungeladen. Bevorzugte Beispiele für ungeladene Aromaten sind Benzol, Toluol, para-Xylol, Hexamethylbenzol und paracymen. Bevorzugte Beispiele für elektrisch geladene Aromaten sind negativ geladene Aromaten, insbesondere Cyclopentadienyl, Indenyl, 4,5-Benzindenyl und Cp* (Pentamethylcyclopentadienyl),
- Olefine, elektrisch neutral oder als Anion, beispielsweise COD (1,5-Cyclooktadienyl), COE (Cyclooctenyl), Allyl oder Methallyl (2-Methylallyl),
- Phosphanen, beispielsweise Mono-, Di- oder Triphosphane, vorzugsweise Monophosphanen, insbesondere tertiären aromatischen Phosphanen, beispielsweise Triphenylphosphan,
- Hydrid und
- Halogeniden, beispielsweise Bromid und insbesondere Chlorid.

Bespiele für als weiterer Ligand geeignete Phosphane sind solche, die mindestens einen unverzweigten oder verzweigten C₁-C₁₂-Alkylrest, mindestens einen C₃-C₁₂-Cycloalkylrest oder mindestens einen aromatischen Rest mit bis zu 24 C-Atomen aufweisen. Beispiele für C₁-C₁₂-Alkylreste sind Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl, 1-Undecyl, 1-Dodecyl, 1-(2-Methyl)-pentyl, 1-(2-Ethyl)-hexyl, 1-(2-n-Propyl)heptyl. Bevorzugte C₁-C₁₂-Alkylreste sind gewählt aus Ethyl, 1-Butyl, sec.-Butyl und 1-Octyl.

Beispiele für C₃-C₁₂-Cycloalkylreste sind insbesondere gewählt aus C₄-C₈-Cycloalkylreste, verzweigt oder unverzweigt, wie Cyclopentyl, Cyclohexyl, Cycloheptyl, Cyclooctyl, Methylcyclopentyl, beispielsweise 2-Methylcyclopentyl, 3-Methylcyclopentyl, weiterhin 2,5-Dimethylcyclopentyl (syn, anti oder als Isomerengemisch), 2-Methylcyclohexyl, 3-Methylcyclohexyl, 4-Methylcyclohexyl, 2,6-Dimethylcyclohexyl (syn, anti oder als Isomerengemisch), Norbonyl und - CH₂-C₆H₁₁. Bevorzugter C₃-C₁₂-Cycloalkylrest ist Cyclohexyl.

In einer bevorzugten Variante wählt man als weiteren Ligand ein Phosphan, das zwei, besonders bevorzugt drei gleiche Reste trägt, beispielsweise Tri-n-butylphosphan, Tri-sec.-butylphosphan, Tricyclohexylphosphan oder Tri-n-octylphosphan.

In einer Ausführungsform wählt man als Substituenten von als weiterem Ligand geeignetem Phosphan mindestens einen aromatischen Rest, beispielsweise 9-Anthracenyl, bevorzugt drei gleiche aromatische Reste, beispielsweise Phenyl, 2-Tolyl, 3-Tolyl, para-Tolyl, Xylyl, 1-Naphthyl, 2-Naphthyl, 1-Binaphthyl, para-Anisyl, 2-Ethylphenyl, 3-Ethylphenyl, para-Ethylphenyl, 2-Chlorphenyl, para-Chlorphenyl, 2,6-Dichlorphenyl, oder mindestens einen heteroaromatischen Rest. Beispiele für heteroaromatische Reste sind Thienyl, Benzothienyl, 1-Naphthothienyl, Thianthrenyl, Furyl, Benzofuryl, Pyrrolyl, Imidazolyl, Pyrazolyl, Pyridyl, Pyrazinyl, Pyrimidinyl, Pyridazinyl, Indolyl, Isoindolyl, Indazolyl, Purinyl, Isoquinolinyl, Quinolinyl, Acridinyl, Naphthyridinyl, Quinoxalinyl, Quinazolinyl, Cinnolinyl, Piperidinyl, Carbolinyl, Thiazolyl, Oxazolyl, Isothiazolyl, Isoxazolyl. Die Heteroarylgruppen können unsubstituiert oder substituiert sein, mit einem oder mehreren Substituenten, die vorstehend unter C₁-C₁₂-Alkyl definiert sind.

In einer anderen bevorzugten Variante wählt man als weiteren Ligand ein mehrzähniges Phosphan, beispielsweise eines mit der Gruppierung >P-CH₂-CH₂-P(C₁-C₁₀-Alkyl)-CH₂CH₂-P<, besonders bevorzugt mit der Gruppierung P-CH₂-CH₂-P<. Beispiel ist 1,2-Bis(dicyclohexylphosphino)ethan.

Es wurde nun beobachtet, dass die bei der Guerbet-Reaktion entstehenden Nebenprodukte, beispielsweise mindestens eine Säure der allgemeinen Formel (III)

R¹-CH₂-COOH (III)

in Form eines ihrer Salze, beispielsweise in Form ihrer Ammoniumsalze oder bevorzugt Alkalimetallsalze, besonders bevorzugt in Form ihrer Natrium- oder Kaliumsalze, wegen ihres hohen Siedepunktes im Reaktionsgefäß verbleiben. Das Gemisch, das man erfindungsgemäß aufarbeitet, enthält also mindestens eine Säure der allgemeinen Formel (III) in Form eines oder mehrerer ihrer Salze.

Dabei ist R¹ wie vorstehend definiert, und R¹ in Säure der allgemeinen Formel (III) entspricht R¹ in Alkohol der Formel (I).

In einer Ausführungsform der vorliegenden Erfindung kann das Gemisch, das man erfindungsgemäß aufarbeitet, mindestens einen verzweigten Alkohol der allgemeinen Formel (II) enthalten.

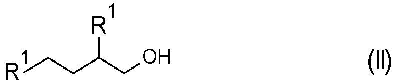

Dabei ist R¹ wie vorstehend definiert und entspricht jeweils R¹ in Alkohol(en) der Formel (I).

In einer Ausführungsform der vorliegenden Erfindung kann das Gemisch, das man erfindungsgemäß aufarbeitet, mindestens einen Ester der allgemeinen Formel (IV) enthalten

R¹-CH₂-COO-CH₂-CH₂-R¹ (IV)

Erfindungsgemäß aufzuarbeitendes Gemisch kann mindestens ein Lösungsmittel enthalten, das von Alkohol der allgemeinen Formel (I) verschieden ist, beispielsweise ein aromatisches Lösungsmittel wie beispielsweise para-Xylol, ortho-Xylol, meta-Xylol, Isomerengemische von Xylol, Mesitylen, oder Toluol, Ethylbenzol oder aliphatische oder cycloaliphatische Lösungsmittel wie beispielsweise n-Hexan, n-Heptan, n-Oktan, n-Nonan, n-Dodecan oder Dekalin. In einer bevorzugten Ausführungsform der vorliegenden Erfindung enthält das Gemisch kein Lösungsmittel, das von Alkohol der allgemeinen Formel (I) und verzweigtem Alkohol der allgemeinen Formel (II) verschieden ist.

In einer Ausführungsform der vorliegenden Erfindung geht man von einem Gemisch aus, das Säure der allgemeinen Formel (III) einerseits und Alkohol der allgemeinen Formel (I) und/oder verzweigter Alkohol der allgemeinen Formel (II) andererseits in einem Gewichtsverhältnis im Bereich von 1 : 100 bis 1 : 1, bevorzugt 1 : 5 bis 1 : 1,05, aufweisen, das heißt Säure (III) zu Summe (Gewicht) aus Alkohol (I) und/oder verzweigtem Alkohol (II) ist im Bereich von 1 : 100 bis 1 : 1, bevorzugt 1 : 5 bis 1 : 1,05.

In einer Ausführungsform der vorliegenden Erfindung geht man von einem Gemisch aus, das eine Suspension ist. So ist es beispielsweise möglich, dass Salz von Säure der allgemeinen Formel (III) als fester Niederschlag vorliegt. In einer anderen Ausführungsform geht man von einem Gemisch aus, das eine mit bloßem Auge homogen aussehende Lösung ist.

Erfindungsgemäß behandelt man vorstehend beschriebenes Gemisch in einem Schritt (a) zunächst mit Wasser, das ein oder mehrere Alkalimetallhydroxide enthalten kann, beispielsweise Natriumhydroxid oder Kaliumhydroxid. In einer anderen Variante behandelt man mit Wasser, das entsalzt ist, oder - in einer weiteren Variante - mit Wasser mit einem neutralem pH-Wert, das aber Salze wie beispielsweise Natriumchlorid enthalten kann.

Zur Behandlung nach Schritt (a) versetzt man zu behandelndes Gemisch mit Wasser und vermischt, beispielsweise über einen Zeitraum von 5 Sekunden bis zu einer halben Stunde. Man kann beispielsweise durch Schütteln vermischen oder vorzugsweise durch Rühren.

In einer Ausführungsform der vorliegenden Erfindung versetzt man in Schritt (a) mit Wasser und mit Alkohol der allgemeinen Formel (I), beispielsweise in einem Volumenverhältnis im Bereich von 5 : 1 bis 1 : 5.

In einer Ausführungsform der vorliegenden Erfindung setzt man in Schritt (a) Wasser einerseits und Gemisch, enthaltend Alkohol der allgemeinen Formel (I) oder verzweigten Alkohol der allgemeinen Formel (II), weiterhin öllösliche Komplexverbindung und Säure der Formel (III) andererseits in einem Volumenverhältnis im Bereich von 10 : 1 bis 1 : 10 ein, bevorzugt in einem Volumenverhältnis im Bereich von 5 : 1 bis 1 : 5.

In einer bevorzugten Ausführungsform der vorliegenden Erfindung setzt man in Schritt (a) Wasser und Gemisch, enthaltend Alkohol der allgemeinen Formel (I) oder verzweigten Alkohol der allgemeinen Formel (II), weiterhin öllösliche Komplexverbindung, und Säure der Formel (III) in einem Volumenverhältnis im Bereich von 0,5 zu 1 bis 2 : 1 ein.

In Schritt (a) bildet sich in vielen Fällen keine homogene Mischung, man beobachtet vielmehr, dass sich zwei verschiedene flüssige Phasen trennen. Dabei ist die organische Phase in vielen Fällen farbig.

In anderen Fällen bildet sich in Schritt (a) eine homogene Phase, die man durch Zugabe von Wasser oder von weiterem Alkohol der allgemeinen Formel (I) in ein Zweiphasengemisch überführen kann.

In Schritt (b) extrahiert man das oder die Salze von Säure der allgemeinen Formel (III). Darunter soll verstanden werden, dass man die Phasentrennung von wässriger Phase, in der sich das oder die Salze von Säure der allgemeinen Formel (III) angereichert haben, abwartet und dann die wässrige Phase abtrennt, beispielsweise über einen Schütteltrichter (Labor) oder über das Bodenablassventil in einem größeren Gefäß, beispielsweise einem Rührkessel oder Autoklaven.

In einer Ausführungsform der vorliegenden Erfindung weist Salz von Säure der allgemeinen Formel (III) in Mischungen von Wasser und Alkohol der allgemeinen Formel (I) bzw. Wasser und verzweigtem Alkohol der allgemeinen Formel (II) einen Nernst'schen Verteilungskoeffizienten im Bereich von 100 bis 3 auf, bestimmt bei Zimmertemperatur.

In einer Ausführungsform der vorliegenden Erfindung wiederholt man die Schritte (a) und (b) ein- oder mehrfach, zum Beispiel dreimal. In einer anderen Ausführungsform der vorliegenden Erfindung wendet man die Schritte (a) und (b) nur jeweils einmal an, bevor man die eigentliche Guerbet-Reaktion wieder startet.

In einer Ausführungsform der vorliegenden Erfindung führt man das erfindungsgemäße Verfahren bei einer Temperatur im Bereich von 5 bis 60 °C, bevorzugt bis 40°C durch. Dabei wird auf die Temperatur nach Schritt (a) Bezug genommen.

In einer Ausführungsform der vorliegenden Erfindung kann man in Schritt (a) von einem Gemisch ausgehen, welches eine Temperatur oberhalb von 60°C aufweist, beispielsweise bis zu 85°C oder bis zu 70°C, und kühlt dieses Gemisch durch Zugabe von Wasser, welches eine Temperatur im Bereich von 10 bis 20°C hat, auf eine Temperatur von 60°C oder niedriger herunter.

In einer Ausführungsform der vorliegenden Erfindung gibt man vor oder nach oder zusammen mit der Zugabe von Wasser in Schritt (a) auch Alkohol der allgemeinen Formel (I) zu. Dabei geht man vorzugsweise so vor, dass das Volumenverhältnis von organischer Phase zu wässriger Phase, also von Gemisch, enthaltend Alkohol der allgemeinen Formel (I) und/oder verzweigten Alkohol der allgemeinen Formel (II), öllösliche Komplexverbindung, in einem Volumenverhältnis im Bereich von 5 zu 1 bis 1 : 5 einstellt.

In einer Ausführungsform der vorliegenden Erfindung führt man die Schritte (a) und (b) des erfindungsgemäßen Verfahrens bei Normaldruck durch. In einer anderen Ausführungsform der vorliegenden Erfindung führt man mindestens einen der Schritte (a) und (b) bei erhöhtem Druck durch, beispielsweise kann man Schritt (b) bei einem Druck im Bereich von 1,1 bis 5 bar durchführen.

Durch die Ausführung des erfindungsgemäßen Verfahrens lassen sich Nebenprodukte der Guerbet-Reaktion und insbesondere Säure der allgemeinen Formel (III) in Form ihrer Salze leicht und schnell aus dem aufzuarbeitenden Gemisch entfernen. Man erhält ein Gemisch, welches Komplex fast quantitativ und in aktiver Form enthält und sich wieder gut durchmischen lässt. Durch Zugabe von Alkohol der allgemeinen Formel (I) und gegebenenfalls von Base startet man die Guerbet-Reaktion zur Herstellung von verzweigtem Alkohol der allgemeinen Formel (II) wieder.

In einer Ausführungsform der vorliegenden Erfindung destilliert man, bevor man das nach dem erfindungsgemäßen Verfahren aufgearbeitete Gemisch wieder für eine Guerbet-Reaktion einsetzt, das durch die Schritte (a) und (b) und in der organischen Phase verbliebene Wasser zunächst zumindest partiell ab, beispielsweise durch azeotrope Destillation mit Alkohol der allgemeinen Formel (I), und gibt erst danach Alkohol der allgemeinen Formel (I) und gegebenenfalls Base zu, um die Guerbet-Reaktion wieder zu starten.

Ein weiterer Gegenstand der vorliegenden Erfindung ist ein Verfahren zur Herstellung von verzweigten Alkoholen der allgemeinen Formel (II)

R¹-CH₂-CH₂-CHR¹-CH₂-OH (II),

kurz auch erfindungsgemäßes Syntheseverfahren genannt, durch Umsetzung von mindestens einem Alkohol der allgemeinen Formel (I)

R¹-CH₂-CH₂-OH (I)

wobei die Gruppen R¹ verschieden oder vorzugsweise gleich sein können und gewählt sind aus C₂-C₁₀-Alkyl, linear oder verzweigt,

in Gegenwart von mindestens einer öllöslichen Komplexverbindung von mindestens einem Metall der 8., 9. oder 10. Gruppe des Periodensystems der Elemente, dadurch gekennzeichnet, dass man Nebenprodukte, gewählt aus Säure der allgemeinen Formel (III)

R¹-CH₂-COOH (III)

in Form eines ihrer Salze nach dem vorstehend beschriebenem - erfindungsgemäßen - Verfahren abtrennt.

Die Umsetzung nach dem erfindungsgemäßen Syntheseverfahren erfolgt in Gegenwart von mindestens einer Base. Als Basen sind Brönsted-Basen bevorzugt. Als Beispiele für geeignete Basen seien genannt: LiOH, NaOH, KOH, LiH, NaH, KH, Ca(OH)₂, CaH₂, LiAlH₄, NaBH₄, LiBH₄, Na₂CO₃, NaHCO₃, Li₂CO₃, LiHCO₃, K₂CO₃, KHCO₃, K₃PO₄, Na₃PO₄, n-Buthyllithium, tert.-BuLi, Methyllithium, Phenyllithium, Lithiummethanolat, Lithiumethanolat, LiO-n-C₃H₇, LiO-iso-C₃H₇, LiO-n-C₄H₉, LiO-iso-C₄H₉, LiO-n-C₅H₁₁, LiO-iso-C₅H₁₁, LiO-n-C₆H₁₃, LiO-iso-C₆H₁₃, Lithium-n-heptanolat, Lithium-n-oktanolat, Lithium-benzylat, Lithiumphenolat, Kaliummethanolat, Kaliumethanolat, KO-n-C₃H₇, KO-iso-C₃H₇, KO-n-C₄H₉, KO-iso-C₄H₉, KO-tert.-C₄H₉, KO-n-C₅H₁₁, KO-iso-C₅H₁₁, KO-n-C₆H₁₃, KO-iso-C₆H₁₃, Kalium-n-heptanolat, Kalium-n-oktanolat, Kaliumbenzylat, Kaliumphenolat, Natriummethanolat, Natriumethanolat, NaO-n-C₃H₇, NaO-iso-C₃H₇, NaO-n-C₄H₉, NaO-iso-C₄H₉, NaO-tert.-C₄H₉, NaO-n-C₅H₁₁, NaO-iso-C₅H₁₁, NaO-n-C₆H₁₃, NaO-iso-C₆H₁₃, Natrium-n-heptanolat, Natrium-n-oktanolat, Natrium-benzylat, Natriumphenolat, KN(SiMe₃)₂, LiN(SiMe₃)₂, NaN(SiMe₃)₂, NH₃ und Amine der Formel (R⁸)ₐNH₃₋ₐ, wobei a gewählt wird aus 1, 2 und 3, und R⁸ = gleich oder verschieden und unabhängig voneinander unsubstituiertes oder zumindest monosubstituiertes C₁-C₁₀-Alkyl, (-C₁-C₄-Alkyl-P(Phenyl)₂), C₃-C₁₀-Cycloalkyl, C₃-C₁₀-Heterocyclyl, wobei unter C₃-C₁₀-Heterocyclyl solche cyclische Gruppen zu verstehen sind, die 3 bis 10 C-Atome und mindestens ein Heteroatom aufweisen, ausgewählt aus N, O und S, weiterhin C₅-C₁₄-Aryl oder C₅-C₁₀-Heteroaryl, wobei C₅-C₁₀-Heteroaryl mindestens ein Heteroatom aufweist, ausgewählt aus N, O und S.

In einer Ausführungsform der vorliegenden Erfindung setzt man insgesamt 0,01 bis 50 Gew.-% Base ein, bevorzugt 0,5 bis 15 Gew.-%, bezogen auf gesamten eingesetzten Alkohol der Formel (I).

In einer Ausführungsform der vorliegenden Erfindung ist die Reaktionsmischung bei Reaktionstemperatur flüssig. Dabei ist es im Rahmen der vorliegenden Erfindung möglich, dass Alkohol der allgemeinen Formel (I) oder mindestens eines seiner Isomeren zumindest partiell in der Gasphase vorliegt.

In einer Ausführungsform der vorliegenden Erfindung führt man die Umsetzung nach dem erfindungsgemäßen Syntheseverfahren bei einer Temperatur im Bereich von 80 bis 200°C, bevorzugt 100 bis 200°C, besonders bevorzugt im Bereich von 110 bis 170°C durch.

In einer Ausführungsform der vorliegenden Erfindung handelt es sich bei der Katalyse um homogene Katalyse. Unter homogener Katalyse wird verstanden, dass man den Katalysator nicht in auf einem festen Träger abgeschiedener Form einsetzt und dass man keine Emulsion erzeugt, in der die Reaktionspartner miteinander reagieren. Der oder die Katalysatoren sind dabei vollständig oder zumindest überwiegend in der Reaktionsmischung gelöst, beispielsweise zu mindestens 90 mol-%, bevorzugt zu mindestens 95 mol-%, bezogen auf das jeweilige Metall der 8., 9. bzw. 10. Gruppe des Periodensystems der Elemente.

In einer Ausführungsform der vorliegenden Erfindung führt man die Umsetzung nach dem erfindungsgemäßen Syntheseverfahren in Gegenwart von mindestens einem inerten Gas durch. Geeignete inerte Gase sind gewählt aus Stickstoff und Edelgasen, insbesondere Argon. In einer anderen Ausführungsform der vorliegenden Erfindung führt man das erfindungsgemäße Verfahren in Gegenwart von Wasserstoff durch. In einer weiteren Ausführungsform führt man das erfindungsgemäße Verfahren in Gegenwart einer Mischung von Wasserstoff und mindestens einem inerten Gas durch.

In einer Ausführungsform der vorliegenden Erfindung führt man die Umsetzung nach dem erfindungsgemäßen Syntheseverfahren bei einem Druck im Bereich von 0,1 bis 5 MPa absolut durch, welcher der Eigendruck des Lösungsmittels bzw. des Alkohols der allgemeinen Formel (I) bei der Reaktionstemperatur bzw. der Druck eines Gases wie Stickstoff, Argon oder Wasserstoff sein kann. Bevorzugt wird die Umsetzung nach dem erfindungsgemäßen Syntheseverfahren bei einem Gesamtdruck bis 3 MPa absolut, insbesondere bevorzugt bei einem Gesamtdruck von 0,1 bis 1 MPa absolut durchgeführt.

Zur Durchführung des erfindungsgemäßen Syntheseverfahrens kann man beispielsweise so vorgehen, dass man Alkohol der allgemeinen Formel (I) mit Base und mindestens einer öllöslichen Komplexverbindung von mindestens einem Metall der 8., 9. oder 10. Gruppe des Periodensystems der Elemente vermischt.

In einer anderen Ausführungsform der vorliegenden Erfindung erzeugt man den Katalysator in situ. Darunter soll zu verstehen sein, dass man öllöslichen Komplexverbindung von mindestens einem Metall der 8., 9. oder 10. Gruppe des Periodensystems der Elemente, bevorzugt Komplexverbindungen von Ir(I), Ir(III), Ru(0), Ru(II) und Ru(III), besonders bevorzugt Komplexverbindung von Ru(II), die vorzugsweise mindestens einen Liganden L¹ aufweist, nicht isoliert, sondern ohne weitere Aufarbeitung durch Vermischen einer Verbindung eines Metalls der 8., 9. oder 10. Gruppe des Periodensystems der Elemente in situ erzeugt, gegebenenfalls in Gegenwart von einem Reduktionsmittel.

Ganz besonders bevorzugt ist es, dass man den Katalysator durch Vermischen von Ir(I), Ir(III), Ru(0), Ru(II)- oder Ru(III)-Ausgangsverbindung und Ligand L¹ erzeugt, beispielsweise indem man Ir(I)-, Ir(III)-, Ru(0-), Ru(II)- oder Ru(III)-Ausgangsverbindung und Ligand L¹ mit Base und Alkohol der allgemeinen Formel (I) vermischt, gegebenenfalls in Gegenwart von einem Reduktionsmittel.

Geeignete Iridium-Ausgangsverbindungen sind beispielsweise IrCl₃, IrCl₃*H₂O, [Ir(COD)Cl]₂, [Ir(COE)₂CIl]₂, [Ir(C₂H₄)₂Cl]₂ [Ir(COD)OH]₂, [Ir(COD)MeO]₂, [IrCp*Cl₂], [IrCpCl₂], Ir₄(CO)₁₂, [Or(PPh₃)₂(CO)Cl], [Ir(acetylacetonat)₃], und [Ir(acetylacetonat)(COD)].

Geeignete Ruthenium-Ausgangsverbindungen sind beispielsweise Ru(p-Cymol)Cl₂]₂, [Ru(benzol)Cl₂]_{y}, [Ru(CO)₂Cl₂]_{y}, wobei y jeweils im Bereich von 1 bis 1.000 liegt, [Ru(CO)₃Cl₂]₂, [Ru(COD)(allyl)], RuCl₃·H₂O, [Ru(acetylacetonat)₃], [Ru(DMSO)₄Cl₂], [Ru(cyclopentadienyl)(CO)₂Cl], [Ru(cyclopentadienyl)(CO)₂H], [Ru(cyclopentadienyl)(CO)₂]₂, [Ru(Cp)(CO)₂Cl], [Ru(Cp*)(CO)₂H], [Ru(Cp*)(CO)₂]₂, [Ru(indenyl)(CO)₂Cl], [Ru(indenyl)(CO)₂H], [Ru(indenyl)(CO)₂]₂, Ruthenocen, [Ru(COD)Cl₂]₂, [Ru(Cp*)(COD)Cl], [RU₃(CO)₁₂], [Ru(PPh₃)₄(H)₂], [Ru(PPh₃)₃(Cl)₂], [Ru(PPh₃)₃(CO)(Cl)₂], [Ru(PPh₃)₃(CO)(Cl)(H)], [Ru(PPh₃)₃(CO)(H)₂] und [Ru(cyclooctadienyl)(methylallyl)₂].

Dabei bedeuten Cp*: Pentamethylcyclopentadienyl, COD: 1,5-Cyclooktadienyl, methylallyl: 2-Methylallyl.

Durch die Wahl der Verbindung eines Metalls der 8., 9. oder 10. Gruppe des Periodensystems der Elemente und insbesondere durch die Wahl der Ir(I)-, Ir(III)-, Ru(0), Ru(II)- oder Ru(III)-Ausgangsverbindung kann man die Auswahl des oder der weiteren Liganden beeinflussen.

In einer Ausführungsform der vorliegenden Erfindung kann man Ligand L¹ und Verbindung des betreffenden Metalls der 8., 9. oder 10. Gruppe des Periodensystems der Elemente und insbesondere die Ir(I)-, Ir(III)-, Ru(0), Ru(II)- oder Ru(III)-Ausgangsverbindung in stöchiometrischen Anteilen, jeweils bezogen auf Zentralmetall, einsetzen. In einer anderen Variante kann man einen Überschuss an Ligand L¹ einsetzen, bezogen auf Metall der 8., 9. bzw. 10. Gruppe des Periodensystems der Elemente, beispielsweise 1,1 bis 5 Moläquivalente L¹ pro Metall der 8., 9. oder 10. Gruppe des Periodensystems der Elemente.

In einer Ausführungsform der vorliegenden Erfindung setzt man 0,001 bis 5 mol-% Ru(II) ein, bezogen auf Alkohol der allgemeinen Formel (I).

Bei der Umsetzung nach dem erfindungsgemäßen Syntheseverfahren wird *in situ* als Nebenprodukt Wasser gebildet. Es ist bevorzugt, das entstehende Wasser, kurz auch Reaktionswasser genannt, abzutrennen.

In einer Ausführungsform der vorliegenden Erfindung trennt man das Reaktionswasser ab, indem man es mit einem azeotropen Schleppmittel abtrennt, beispielsweise einem der vorstehend genannten Lösungsmittel, insbesondere einem der vorstehend genannten aromatischen Lösungsmittel. In einer bevorzugten Variante geht man so vor, dass man Alkohol der allgemeinen Formel (I) als azeotropes Schleppmittel einsetzt, da er mit Wasser eine Mischungslücke aufweist, um Reaktionswasser abzutrennen bzw. auszukreisen.

Bevorzugt wird während der Umsetzung das Reaktionswasser mit Hilfe eines Wasserabscheiders ausgekreist.

Das erfindungsgemäße Syntheseverfahren kann man in verschiedensten Reaktionsgefäßen durchführen, in denen Flüssigkeitsreaktionen, gegebenenfalls mit einem Gasraum, durchgeführt werden können. Geeignete Reaktionsgefäße sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt: Rührkesselreaktoren, Rohrreaktoren und Blasensäulenreaktoren.

Das erfindungsgemäße Syntheseverfahren kann man diskontinuierlich, also in Batchfahrweise, oder kontinuierlich oder semikontinuierlich mit Rückführung oder ohne Rückführung durchgeführt werden. Die mittlere Verweilzeit der entstehenden Reaktionsmasse im Reaktionsgefäß kann beispielsweise im Bereich von 15 Minuten bis 100 Stunden betragen.

In einer Ausführungsform der vorliegenden Erfindung führt man das erfindungsgemäße Verfahren bis zu vollständigem Umsatz von Alkohol der allgemeinen Formel (I) durch. In einer anderen Ausführungsform führt man die Umsetzung nur zu unvollständigem Umsatz durch, beispielsweise zu 8 bis 50 mol-%, bevorzugt bis 30 mol-%, und arbeitet danach auf.

Ohne dass einer bestimmten Theorie der Vorzug gegeben werden soll, so ist es plausibel, dass das erfindungsgemäße Verfahren im Wesentlichen drei Reaktionen umfasst. Zunächst wird Alkohol der Formel (I) oxidativ dehydriert, und zwar zum Aldehyd. Dann erfolgt eine Aldolkondensation, gefolgt von einer Reduktion.

Durch die Ausführung des erfindungsgemäßen Verfahrens erhält man verzweigten Alkohol der allgemeinen Formel (II) 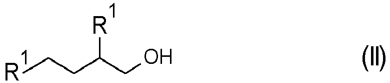 wobei die Gruppen R¹ verschieden sind oder gleich und wie vorstehend definiert.

Wenn man eine Mischung von Alkohol der allgemeinen Formel (I) mit einem oder mehreren Isomeren als Ausgangsmaterial einsetzt, so erhält man üblicherweise ein Gemisch von verzweigten Alkoholen der allgemeinen Formel (II).

In einer Ausführungsform der vorliegenden Erfindung erhält man verzweigten Alkohol der allgemeinen Formel (II.1) 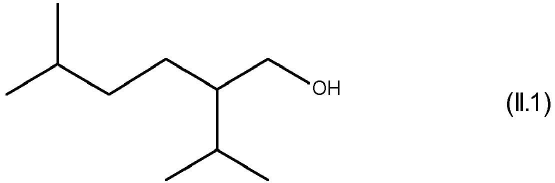 im Gemisch mit Alkohol der Formel (II.1a)

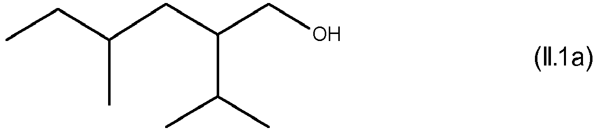

In einer anderen Ausführungsform erhält man verzweigten Alkohol der Formel (II.2) 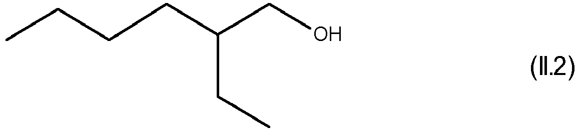 im Gemisch mit Alkohol der Formel (II.2a)

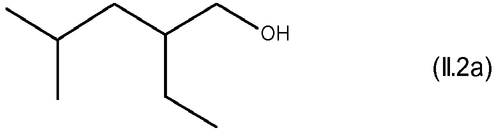

In einer Ausführungsform der vorliegenden Erfindung erhält man als Nebenprodukt Säure der Formel (III), beispielsweise - wenn man von Isoamylalkohol als Alkohol der Formel (I) ausgeht-eine Säure der Formel

(CH₃)₂CH-(CH₂)-COOH.

In einer Ausführungsform der vorliegenden Erfindung erhält man als Nebenprodukt Ester der Formel (IV), beispielsweise - wenn man von Isoamylalkohol als Alkohol der Formel (I) ausgeht - einen Ester der Formel

(CH₃)₂CH-(CH₂)-COO-(CH₂)₂-CH(CH₃)₂

Dieser kann während der Umsetzung verseift werden.

Im Anschluss an die eigentliche Synthese arbeitet man auf. Zum Zweck der Aufarbeitung kann man beispielsweise so vorgehen, dass man verzweigten Alkohol der allgemeinen Formel (II) von nicht umgesetztem Alkohol der allgemeinen Formel (I) sowie von Base und Komplex durch Destillation abtrennt. Komplex und Base verbleibt mit eventuell angefallenen Hochsiedern, beispielsweise Trimerisierungsprodukt von Alkohol der allgemeinen Formel (I), im Sumpf der Destillation zurück und kann wiederverwendet werden. Nicht umgesetzten Alkohol der allgemeinen Formel (I) kann man ebenfalls wieder in die Reaktion zurückführen. Die thermische Abtrennung von verzweigtem Alkohol der allgemeinen Formel (II) sowie von gegebenenfalls gebildetem Ester kann beispielsweise nach an sich bekannten Verfahren erfolgen, bevorzugt in einem Verdampfer oder in einer Destillationseinheit, umfassend Verdampfer und Kolonne(n), die üblicherweise mehrere Böden oder eine Packung oder Füllkörper aufweist bzw. aufweisen.

Durch das erfindungsgemäße Verfahren lassen sich verzweigte Alkohole der Formel (I) in guter Ausbeute und sehr gute Reinheit herstellen. Man kann zu ihrer Herstellung nicht nur von reinem Alkohol der allgemeinen Formel (I) ausgehen, sondern auch Isomerengemische verwenden, beispielsweise solche, die man durch Fermentation oder anderen biologischen Abbau von Sacchariden erhalten kann, insbesondere sogenannte Fuselöle, beispielsweise Fuselalkohol (Isomerengemisch aus 3-Methylbutanol und 2-Methylbutanol).

Die Abfolge von Umsetzung und destillativer Aufarbeitung wird im Rahmen der vorliegenden Erfindung auch Zyklus genannt.

Nach 3 bis 100, insbesondere nach 3 bis 10 Zyklen reichert sich so viel Nebenprodukt an, beispielsweise Säure der allgemeinen Formel (III), dass Umsatz und Ausbeute nachlassen und die Durchmischbarkeit stark abnimmt. Vorzugsweise nach 3 bis 100 Zyklen führt man demnach das eingangs beschriebene erfindungsgemäße Verfahren durch. Dadurch wird Säure der allgemeinen Formel (III) weitgehend, und zwar als Salz, beispielsweise zu mindestens 80 mol-%, oder vorzugsweise vollkommen entfernt, und man kann mit dem erfindungsgemäßen Syntheseverfahren fortfahren, beispielsweise durch Zugabe von Alkohol der Formel (I), gegebenenfalls von Base, und durch Erwärmen.

In einer Ausführungsform der vorliegenden Erfindung destilliert man, bevor mit dem erfindungsgemäßen Syntheseverfahren fortfährt, das durch die Schritte (a) und (b) und in der organischen Phase verbliebene Wasser zunächst zumindest partiell ab, beispielsweise durch azeotrope Destillation mit Alkohol der allgemeinen Formel (I), und gibt erst danach Alkohol der allgemeinen Formel (I) und gegebenenfalls Base zu, um die Guerbet-Reaktion wieder zu starten.

Die Erfindung wird durch ein Arbeitsbeispiel weiter erläutert.

### Arbeitsbeispiel:

### Allgemeine Arbeitsvorschrift:

Die Umsetzung wurde unter Inertbedingungen (Argonüberdeckung) in einem 4-l-Dreihalskolben mit Rührer und Rückflusskühler durchgeführt. Man legte 2,240 kg Fuselalkohol (Isomerengemisch aus 3-Methylbutanol und 2-Methylbutanol, Molverhältnis 80:20) vor und gab unter Rühren 130 g KOH zu. Man rührte 10 Minuten bei Zimmertemperatur und gab dann 9,7 g Triphenylphosphan zu. Man rührte weitere 10 Minuten bei Zimmertemperatur und gab dann 3,6 g [Ru(COD)Cl₂]₂ zu. Es bildete sich eine hellbraune Suspension. Man überführte die hellbraune Suspension unter Stickstoff in einen mit Stickstoff inertisierten 4-Liter-Doppelmantel-Glasreaktor mit Wasserabscheider (Wasserauskreiser) und Schrägblattrührer und rührte 2 Stunden bei 100°C. Danach gab man eine Lösung von 3,4 g Bis-2,6-(diethylamino)lutidin zu, siehe Formel, 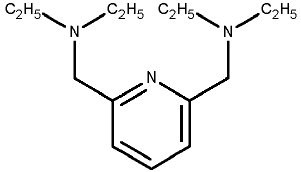 gelöst in 60 g Fuselalkohol (Isomerengemisch aus 3-Methylbutanol und 2-Methylbutanol, Molverhältnis 80:20). Man erwärmte auf 170°C (Manteltemperatur) und rührte unter Wasserauskreisung über einen Zeitraum von 16 Stunden. Dann ersetzte man den Wasserauskreiser durch eine Destillationskolonne und destillierte nicht umgesetzten Fuselalkohol (bei 4 mbar_{absolut} und 60°C) und Verbindung (II.1) 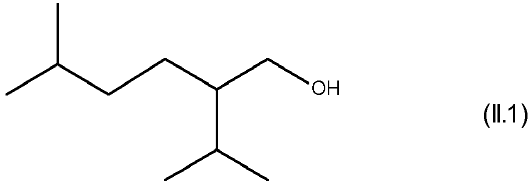 und Rohprodukt, enthaltend (II.1a) 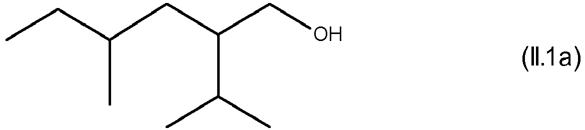 bei 3 mbar_{absolut} und 110°C ab. Man ließ auf Zimmertemperatur abkühlen und stellte durch Stickstoff Normaldruck ein. Damit war der 1. Zyklus abgeschlossen.

In den folgenden Zyklen befüllte man jeweils mit Fuselalkohol und KOH nach Tabelle 1 und wiederholte den ersten Zyklus.

Die Ergebnisse der folgenden Zyklen sind in Tabelle 1 zusammengefasst.

**Tabelle 1: Resultate der Zyklen 1 bis 4**

| Zyklus | zugesetzter Fuselalkohol | Zugesetzte KOH | Ausgekreiste Wassermenge | Abdestillierter Fuselalkohol | Abdestilliertes Rohprodukt [(II.1) + (II.1a)] |
|---|---|---|---|---|---|
| 1 | 2300 g | 130 g | 150 ml | 924 g | 680 g |
| 2 | 1820 g | 140 g | 150 ml | 311 g | 1070 g |
| 3 | 1805 g | 140 g | 150 ml | 480 g | 990 g |
| 4 | 1815 g | 170 g | 170 ml | 167 g | 994 g |

Nach dem Ende des 4. Zyklus befand sich im Destillationsrückstand so viel Feststoff im Glasreaktor, dass der Rührer stark behindert wurde und sich der Feststoff nicht mehr vollständig in Fuselöl löste. Man mischte den Destillationsrückstand mit 2,5 Liter Wasser und 1 Liter Fuselöl. Man erhielt eine einphasige Lösung, die man auf drei gleich große Portionen aufteilte, die man jeweils mit 0,5 Liter Wasser extrahierte. Man erhielt insgesamt 4 Liter wässrige Phase (enthaltend 4 ppm Ru-Verbindungen) und insgesamt etwa einen Liter organische Phase (enthaltend 520 ppm Ru-Verbindungen). Man füllte die organische Phase wieder in den Glasreaktor, versetzte mit 140 g KOH und einem Liter Fuselöl und begann so den 5. Zyklus. Die Ergebnisse der Zyklen 5 und 6 sind in Tabelle 2 dargestellt.

**Tabelle 2: Resultate der Zyklen 5 und 6**

| Zyklus | zugesetzter Fuselalkohol | Zugesetzte KOH | Ausgekreiste Wassermenge | Abdestillierter Fuselalkohol | Abdestilliertes Rohprodukt [(II.1) + (II.1a)] |
|---|---|---|---|---|---|
| 5 | 1000 g | 140 g | 85 ml | 523 g | 567 g |
| 6 | 2000 g | 180 g | 100 ml | 407 g | 792 g |

## Patentansprüche

1. Verfahren zur Herstellung eines verzweigten Alkohols der allgemeinen Formel (II)
R¹-CH₂-CH₂-CHR¹-CH₂-OH (II)
aus mindestens einem Alkohol der Formel (I)
R¹-CH₂-CH₂-OH (I),
weiterhin mindestens einer Komplexverbindung von mindestens einem Metall der 8., 9. oder 10. Gruppe des Periodensystems der Elemente, die gewählt ist aus Komplexverbindungen, die mindestens einen Liganden L¹ haben, der mindestens zweizähnig ist, wobei mindestens eine Koordinationsstelle von L¹ ein Stickstoffatom ist, und wobei die Komplexverbindung in mindestens einem der Lösungsmittel Toluol, m-Xylol, n-Hexan oder Alkohol der allgemeinen Formel (I) oder verzweigtem Alkohol der allgemeinen Formel (II) eine Löslichkeit von mindestens 0,5 g/l aufweist, gemessen bei Zimmertemperatur,
wobei man das durch die Reaktion erhältliche Gemisch, das mindestens den Alkohol der allgemeinen Formel (I) oder mindestens den verzweigten Alkohol der allgemeinen Formel (II) und weiterhin die Komplexverbindung und mindestens eine Säure der allgemeinen Formel (III)
R¹-CH₂-COOH (III)
in Form eines ihrer Salze enthält,
wobei die Gruppen R¹ gewählt sind aus C₂-C₁₀-Alkyl, linear oder verzweigt,
aufarbeitet, indem man
(a) das Gemisch mit Wasser behandelt, das ein Alkalimetallhydroxid enthalten kann,
(b) das oder die Salze von Säure der allgemeinen Formel (III) extrahiert,
und dass man das so erhältliche Gemisch einsetzt, um durch Zugabe von Alkohol der allgemeinen Formel (I) und gegebenenfalls von Base die Guerbet-Reaktion zur Herstellung von verzweigtem Alkohol der allgemeinen Formel (II) wieder zu starten.

2. Verfahren nach Anspruch 1, **dadurch gekennzeichnet, dass** das Gemisch sowohl mindestens einen Alkohol der allgemeinen Formel (I) als auch mindestens einen verzweigten Alkohol der allgemeinen Formel (II) enthält.

3. Verfahren nach Anspruch 1 oder 2, **dadurch gekennzeichnet, dass** man es bei Temperaturen im Bereich von 5 bis 60°C durchführt.

4. Verfahren nach einem der Ansprüche 1 bis 3, **dadurch gekennzeichnet, dass** man R¹ wählt aus Ethyl, n-Propyl, n-Butyl und Isopropyl.

5. Verfahren nach einem der Ansprüche 1 bis 4, **dadurch gekennzeichnet, dass** man öllösliche Komplexverbindung wählt aus Komplexverbindungen von Ir(I), Ir(III), Ru(0), Ru(II) und Ru(III).

6. Verfahren nach einem der Ansprüche 1 bis 5, **dadurch gekennzeichnet, dass** man L¹ wählt aus zweizähnigen und dreizähnigen Liganden, die über ein oder mehrere Stickstoffatome und gegebenenfalls ein oder mehr Carben-Kohlenstoffatome mit dem Zentralmetall koordinieren.

7. Verfahren nach einem der Ansprüche 1 bis 6, **dadurch gekennzeichnet, dass** öllösliche Komplexverbindung mindestens einen weiteren Liganden aufweist, gewählt aus CO, Pseudohalogeniden, organischen Carbonylverbindungen, Aromaten, Olefinen, Phosphanen, Hydrid und Halogeniden.

8. Verfahren nach einem der Ansprüche 1 bis 7, **dadurch gekennzeichnet, dass** man L¹ wählt aus Verbindungen der allgemeinen Formel (V) 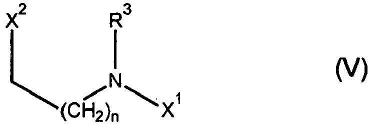 wobei die Variablen wie folgt gewählt sind:
R³ gewählt aus Wasserstoff und C₁-C₅-Alkyl,
n gewählt aus null und eins,
X¹ gewählt aus Wasserstoff, C₁-C₅-Alkyl und (CH₂)ₙ₊₁-X²,
X² gewählt aus NR⁴R⁵, 2-Pyridyl und Imidazol-2-ylidenyl der Formel 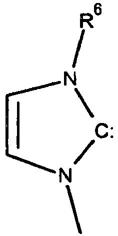
R⁴, R⁵ verschieden oder gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.

9. Verfahren nach einem der Ansprüche 1 bis 7, **dadurch gekennzeichnet, dass** man L¹ wählt aus Verbindungen der allgemeinen Formel (VI) 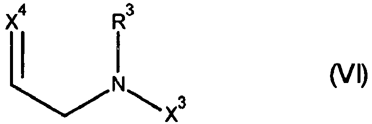 wobei die Variablen wie folgt gewählt sind:
R³ gewählt aus Wasserstoff, C₁-C₁₀-Alkyl, C₃-C₁₀-Cycloalkyl, Benzyl und Phenyl,
X³ gewählt aus Wasserstoff, C₁-C₅-Alkyl und CH₂=X⁴,
X⁴ gewählt aus NR⁴, und
R⁴ gewählt aus C₁-C₁₀-Alkyl und C₃-C₁₀-Cycloalkyl, Benzyl und Phenyl, unsubstituiert oder ein- oder mehrfach substituiert mit C₁-C₃-Alkyl.

10. Verfahren nach einem der Ansprüche 1 bis 7, **dadurch gekennzeichnet, dass** man L¹ wählt aus Verbindungen der allgemeinen Formel (VII) 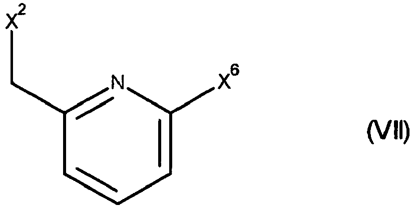 wobei die Variablen wie folgt gewählt sind:
X⁶ gewählt aus Wasserstoff, C₁-C₅-Alkyl und CH₂-X²,
X² gewählt aus NR⁴R⁵, 2-Pyridyl und Imidazol-2-ylidenyl der Formel 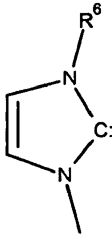
R⁴, R⁵ verschieden oder gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.

11. Verfahren nach einem der Ansprüche 1 bis 7, **dadurch gekennzeichnet, dass** man L¹ wählt aus Verbindungen der allgemeinen Formel (VIII) 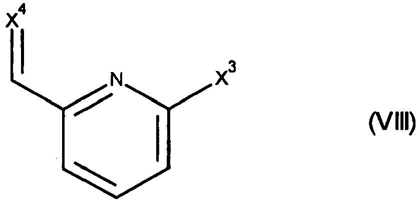 wobei die Variablen wie folgt gewählt sind:
X³ gewählt aus Wasserstoff, C₁-C₅-Alkyl und CH₂=X⁴,
X⁴ verschieden oder gleich und gewählt aus N-R⁴,
R⁴ gewählt aus C₁-C₁₀-Alkyl, C₃-C₈-Cycloalkyl, Benzyl und Phenyl, unsubstituiert oder ein- oder mehrfach substituiert mit C₁-C₃-Alkyl.

12. Verfahren nach einem der Ansprüche 1 bis 11, **dadurch gekennzeichnet, dass** man L¹ wählt aus Verbindungen der Formel (IX) 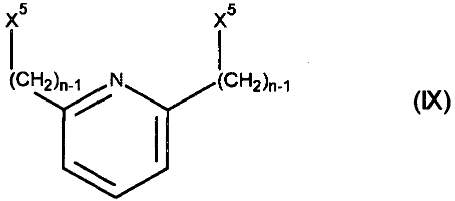 wobei die Variablen wie folgt gewählt sind:
n gleich oder verschieden und jeweils null oder eins
X⁵ jeweils gleich und gewählt aus NR⁴R⁵, 2-Pyridyl und Imidazol-2-ylidenyl der Formel 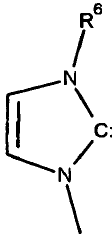
R⁴, R⁵ verschieden oder gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.

13. Verfahren nach einem der Ansprüche 1 bis 12, **dadurch gekennzeichnet, dass** man in Schritt (a) Wasser und Gemisch, enthaltend Alkohol der allgemeinen Formel (I) und/oder verzweigten Alkohol der Formel (II), weiterhin öllösliche Komplexverbindung, und Säure der Formel (III) in einem Volumenverhältnis im Bereich von 0,5 zu 1 bis 2 : 1 einsetzt.

14. Verfahren zur Herstellung von verzweigten Alkoholen der allgemeinen Formel (II),
R¹-CH₂-CH₂-CHR¹-CH₂-OH (II)
durch Umsetzung von mindestens einem Alkohol der allgemeinen Formel (I)
R¹-CH₂-CH₂-OH (I)
wobei die Gruppen R¹ gewählt sind aus C₂-C₁₀-Alkyl, linear oder verzweigt,
in Gegenwart von mindestens einer Komplexverbindung von mindestens einem Metall der 8., 9. oder 10. Gruppe des Periodensystems der Elemente, die gewählt ist aus Komplexverbindungen, die mindestens einen Liganden L¹ haben, der mindestens zweizähnig ist, wobei mindestens eine Koordinationsstelle von L¹ ein Stickstoffatom ist, und wobei die Komplexverbindung in mindestens einem der Lösungsmittel Toluol, m-Xylol, n-Hexan oder Alkohol der allgemeinen Formel (I) oder verzweigtem Alkohol der allgemeinen Formel (II) eine Löslichkeit von mindestens 0,5 g/l aufweist, gemessen bei Zimmertemperatur,
**dadurch gekennzeichnet, dass** man Nebenprodukte, gewählt aus Säuren der allgemeinen Formel (III)
R¹-CH₂-COOH (III)
in Form eines ihrer Salze nach einem Verfahren nach einem der Ansprüche 1 bis 13 abtrennt.

## Claims

1. A method for preparing a branched alcohol of the general formula (II)
R¹-CH₂-CH₂-CHR¹-CH₂-OH (II)
from at least one alcohol of the formula (I)
R¹-CH₂-CH₂-OH (I),
furthermore at least one complex compound of at least one metal of the 8th, 9th or 10th group of the Periodic Table of the Elements , which is selected from complex compounds which have at least one ligand L¹ which is at least bidentate, where at least one coordination site of L¹ is a nitrogen atom, and where the complex compound has a solubility of at least 0.5 g/l, measured at room temperature, in at least one of the solvents toluene, m-xylene, n-hexane or alcohol of the general formula (I) or branched alcohol of the general formula (II),
where the mixture which can be obtained by the reaction and comprises at least the alcohol of the general formula (I) or at least the branched alcohol of the general formula (II) and furthermore the complex compound and at least one acid of the general formula (III)
R¹-CH₂-COOH (III)
in the form of one of its salts,
where the groups R¹ are selected from C₂-C₁₀-alkyl, linear or branched,
is worked up by
(a) treating the mixture with water which can comprise an alkali metal hydroxide,
(b) extracting the salt or salts of acid of the general formula (III),
and the mixture which can be obtained in this way is used, by adding alcohol of the general formula (I) and optionally base, to start the Guerbet reaction again for preparing branched alcohol of the general formula (II).

2. The method according to claim 1, wherein the mixture comprises both at least one alcohol of the general formula (I) and also at least one branched alcohol of the general formula (II).

3. The method according to claim 1 or 2, which is carried out at temperatures in the range from 5 to 60°C.

4. The method according to any one of claims 1 to 3, wherein R¹ is selected from ethyl, n-propyl, n-butyl and isopropyl.

5. The method according to any one of claims 1 to 4, wherein oil-soluble complex compound is selected from complex compounds of Ir(I), Ir(III), Ru(0), Ru (II) and Ru (III).

6. The method according to any one of claims 1 to 5, wherein L¹ is selected from bidentate and tridentate ligands which coordinate with the central metal via one or more nitrogen atoms and optionally one or more carbene carbon atoms.

7. The method according to any one of claims 1 to 6, wherein oil-soluble complex compound has at least one further ligand selected from CO, pseudohalides, organic carbonyl compounds, aromatics, olefins, phosphanes, hydride and halides.

8. The method according to any one of claims 1 to 7, wherein L¹ is selected from compounds of the general formula (V) 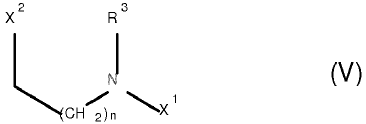 where the variables are selected as follows:
R³ is selected from hydrogen and C₁-C₅-alkyl,
n is selected from zero and one,
X¹ is selected from hydrogen, C₁-C₅-alkyl and (CH₂)ₙ₊₁-X²,
X² is selected from NR⁴R⁵, 2-pyridyl and imidazol-2-ylidenyl of the formula 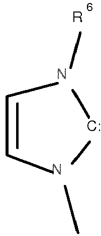
R⁴, R⁵ are identical or different and selected from C₁-C₁₀-alkyl, C₃-C₁₀-cycloalkyl, benzyl and phenyl, unsubstituted or mono- or polysubstituted with C₁-C₃-alkyl,
R⁶ is selected from C₁-C₁₀-alkyl, C₃-C₁₀-cycloalkyl, benzyl and phenyl, unsubstituted or mono- or polysubstituted with C₁-C₃-alkyl.

9. The method according to any one of claims 1 to 7, wherein L¹ is selected from compounds of the general formula (VI) 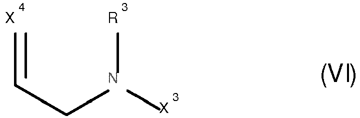 where the variables are selected as follows:
R³ is selected from hydrogen, C₁-C₁₀-alkyl, C₃-C₁₀-cycloalkyl, benzyl and phenyl,
X³ is selected from hydrogen, C₁-C₅-alkyl and CH₂=X⁴,
X⁴ is selected from NR⁴, and
R⁴ is selected from C₁-C₁₀-alkyl and C₃-C₁₀-cycloalkyl, benzyl and phenyl, unsubstituted or mono- or polysubstituted with C₁-C₃-alkyl.

10. The method according to any one of claims 1 to 7, wherein L¹ is selected from compounds of the general formula (VII) 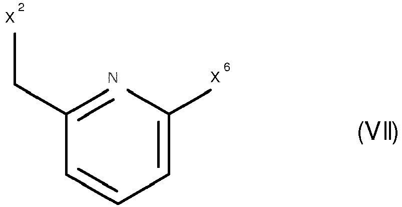 where the variables are selected as follows:
X⁶ is selected from hydrogen, C₁-C₅-alkyl and CH₂-X²,
X² is selected from NR⁴R⁵, 2-pyridyl and imidazol-2-ylidenyl of the formula 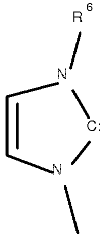
R⁴, R⁵ are different or identical and selected from C₁-C₁₀-alkyl, C₃-C₁₀-cycloalkyl, benzyl and phenyl, unsubstituted or mono- or polysubstituted with C₁-C₃-alkyl,
R⁶ is selected from C₁-Cₗo-alkyl, C₃-C₁₀-cycloalkyl, benzyl and phenyl, unsubstituted or mono- or polysubstituted with C₁-C₃-alkyl.

11. The method according to any one of claims 1 to 7, wherein L¹ is selected from compounds of the general formula (VIII) 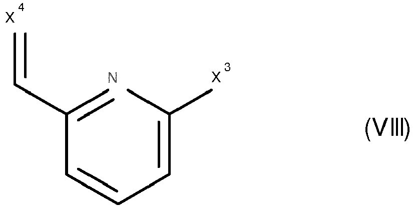 where the variables are selected as follows:
X³ is selected from hydrogen, C₁-C₅-alkyl and CH₂=X⁴,
X⁴ is different or identical and selected from N-R⁴,
R⁴ is selected from C₁-C₁₀-alkyl, C₃-C₈-cycloalkyl, benzyl and phenyl, unsubstituted or mono- or polysubstituted with C₁-C₃-alkyl.

12. The method according to any one of claims 1 to 11, wherein L¹ is selected from compounds of the formula (IX) 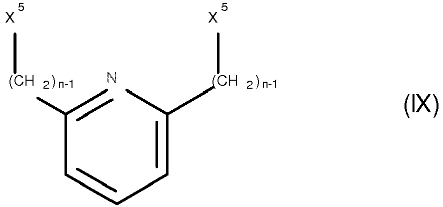 where the variables are selected as follows:
n is identical or different and in each case zero or one
X⁵ is in each case identical and selected from NR⁴R⁵, 2-pyridyl and imidazol-2-ylidenyl of the formula 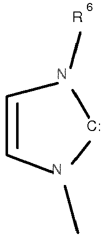
R⁴, R⁵ are different or identical and selected from C₁-C₁₀-alkyl, C₃-C₁₀-cycloalkyl, benzyl and phenyl, unsubstituted or mono- or polysubstituted with C₁-C₃-alkyl,
R⁶ is selected from C₁-C₁₀-alkyl, C₃-C₁₀-cycloalkyl, benzyl and phenyl, unsubstituted or mono- or polysubstituted with C₁-C₃-alkyl.

13. The method according to any one of claims 1 to 12, wherein, in step (a), water and mixture comprising alcohol of the general formula (I) and/or branched alcohol of the formula (II), furthermore oil-soluble complex compound, and acid of the formula (III) are used in a volume ratio in the range from 0.5:1 to 2:1.

14. A method for preparing branched alcohols of the general formula (II),
R¹-CH₂-CH₂-CHR¹-CH₂-OH (II)
by reacting at least one alcohol of the general formula (I)
R¹-CH₂-CH₂-OH (I)
where the groups R¹ are selected from C₂-C₁₀-alkyl, linear or branched,
in the presence of at least one complex compound of at least one metal of the 8th, 9th or 10th group of the Periodic Table of the Elements, which is selected from complex compounds which have at least one ligand L¹ which is at least bidentate, where at least one coordination site of L¹ is a nitrogen atom, and where the complex compound has a solubility of at least 0.5 g/l, measured at room temperature, in at least one of the solvents toluene, m-xylene, n-hexane or alcohol of the general formula (I) or branched alcohol of the general formula (II),
wherein by-products selected from acids of the general formula (III)
R¹-CH₂-COOH (III)
are separated off in the form of one of their salts by a method according to any one of claims 1 to 13.

## Revendications

1. Procédé pour la préparation d'un alcool ramifié de formule générale (II)
R¹-CH₂-CH₂-CHR¹-CH₂-OH (II)
à partir d'au moins un alcool de formule (I)
R¹-CH₂-CH₂-OH (I),
en outre à partir d'au moins un composé complexe d'au moins un métal du 8ème, 9ème ou 10ème groupe du système périodique des éléments, qui est choisi parmi les composés complexes, qui présentent au moins un ligand L¹, qui est au moins bidentate, au moins un site de coordination de L¹ étant un atome d'azote, et le composé complexe présentant une solubilité d'au moins 0,5 g/l, mesurée à température ambiante, dans au moins un des solvants toluène, m-xylène, n-hexane ou alcool de formule générale (I) ou alcool ramifié de formule générale (II), le mélange, pouvant être obtenu par la réaction, qui contient au moins l'alcool de formule générale (I) ou au moins l'alcool ramifié de formule générale (II) et en outre le composé complexe et au moins un acide de formule générale (III)
R¹-CH₂-COOH (III)
sous forme d'un de ses sels, les groupes R¹ étant choisis parmi les radicaux C₂-C₁₀-alkyle linéaires ou ramifiés, étant traité en ce que
(a) on traite le mélange avec de l'eau qui peut contenir un hydroxyde de métal alcalin,
(b) on extrait le ou les sels de l'acide de formule générale (III),
et on utilise le mélange ainsi obtenu pour redémarrer la réaction de Guerbet pour la préparation d'alcool ramifié de formule générale (II) par addition d'alcool de formule générale (I) et le cas échéant de base.

2. Procédé selon la revendication 1, **caractérisé en ce que** le mélange contient au moins un alcool de formule générale (I) ainsi qu'au moins un alcool ramifié de formule générale (II).

3. Procédé selon la revendication 1 ou 2, **caractérisé en ce qu'**on le réalise à des températures dans la plage de 5 à 60°C.

4. Procédé selon l'une quelconque des revendications 1 à 3, **caractérisé en ce qu'**on choisit R¹ parmi éthyle, n-propyle, n-butyle et isopropyle.

5. Procédé selon l'une quelconque des revendications 1 à 4, **caractérisé en ce qu'**on choisit un composé complexe soluble dans l'huile parmi les composés complexes d'Ir(I), d'Ir(III), de Ru(0), de Ru(II) et de Ru(III).

6. Procédé selon l'une quelconque des revendications 1 à 5, **caractérisé en ce qu'**on choisit L¹ parmi les ligands bidentates et tridentates, qui coordinent le métal central via un ou plusieurs atomes d'azote et le cas échéant un ou plusieurs atomes de carbone à fonction carbène.

7. Procédé selon l'une quelconque des revendications 1 à 6, **caractérisé en ce que** le composé complexe soluble dans l'huile présente au moins un autre ligand, choisi parmi CO, les pseudohalogénures, les composés carbonyle organiques, les aromatiques, les oléfines, les phosphanes, l'hydrure et les halogénures.

8. Procédé selon l'une quelconque des revendications 1 à 7, **caractérisé en ce qu'**on choisit L¹ parmi les composés de formule générale (V) 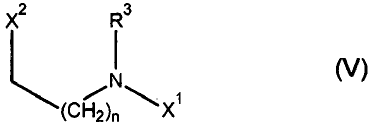 les variables étant choisies comme suit :
R³ choisi parmi hydrogène et C₁-C₅-alkyle,
n choisi entre zéro et un,
X¹ choisi parmi hydrogène, C₁-C₅-alkyle et (CH₂)ₙ₊₁-X²,
X² choisi parmi NR⁴R⁵, 2-pyridyle et imidazol-2-ylidényle de formule 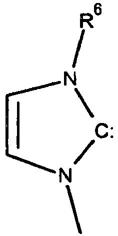
R⁴, R⁵ identiques ou différents, choisis parmi C₁-C₁₀-alkyle, C₃-C₁₀-cycloalkyle, benzyle et phényle, non substitué ou monosubstitué ou polysubstitué par C₁-C₃-alkyle,
R⁶ choisi parmi C₁-C₁₀-alkyle, C₃-C₁₀-cycloalkyle, benzyle et phényle, non substitué ou monosubstitué ou polysubstitué par C₁-C₃-alkyle.

9. Procédé selon l'une quelconque des revendications 1 à 7, **caractérisé en ce qu'**on choisit L¹ parmi les composés de formule générale (VI) 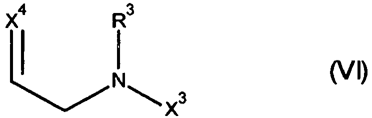 les variables étant choisies comme suit :
R³ choisi parmi hydrogène, C₁-C₁₀-alkyle, C₃-C₁₀-cycloalkyle, benzyle et phényle,
X³ choisi parmi hydrogène, C₁-C₅-alkyle et CH₂=X⁴,
X⁴ choisi parmi NR⁴ et
R⁴ choisi parmi C₁-C₁₀-alkyle et C₃-C₁₀-cycloalkyle, benzyle et phényle, non substitué ou monosubstitué ou polysubstitué par C₁-C₃-alkyle.

10. Procédé selon l'une quelconque des revendications 1 à 7, **caractérisé en ce qu'**on choisit L¹ parmi les composés de formule générale (VII) 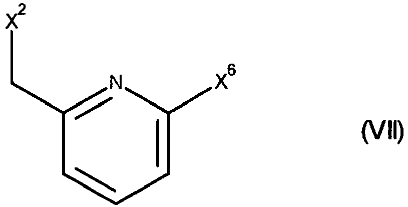 les variables étant choisies comme suit :
X⁶ choisi parmi hydrogène, C₁-C₅-alkyle et CH₂-X²,
X² choisi parmi NR⁴R⁵, 2-pyridyle et imidazol-2-ylidényle de formule 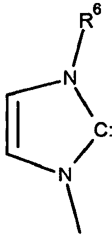
R⁴, R⁵ identiques ou différents, choisis parmi C₁-C₁₀-alkyle, C₃-C₁₀-cycloalkyle, benzyle et phényle, non substitué ou monosubstitué ou polysubstitué par C₁-C₃-alkyle,
R⁶ choisi parmi C₁-C₁₀-alkyle, C₃-C₁₀-cycloalkyle, benzyle et phényle, non substitué ou monosubstitué ou polysubstitué par C₁-C₃-alkyle.

11. Procédé selon l'une quelconque des revendications 1 à 7, **caractérisé en ce qu'**on choisit L¹ parmi les composés de formule générale (VIII) 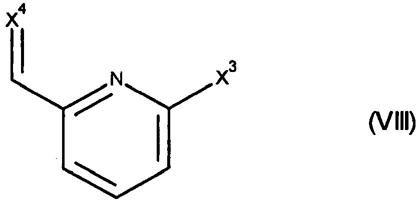 les variables étant choisies comme suit :
X³ choisi parmi hydrogène, C₁-C₅-alkyle et CH₂=X⁴,
X⁴ identique ou différent, choisi parmi N-R⁴,
R⁴ choisi parmi C₁-C₁₀-alkyle, C₃₋C₈-cycloalkyle, benzyle et phényle, non substitué ou monosubstitué ou polysubstitué par C₁-C₃-alkyle.

12. Procédé selon l'une quelconque des revendications 1 à 11, **caractérisé en ce qu'**on choisit L¹ parmi les composés de formule générale (IX) 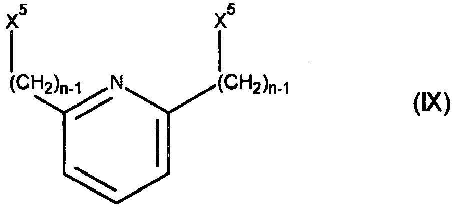 les variables étant choisies comme suit :
n identique ou différent, signifie à chaque fois zéro ou un
X⁵ à chaque fois identique, choisi parmi NR⁴R⁵, 2-pyridyle et imidazol-2-ylidényle de formule 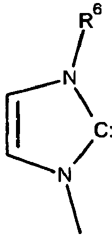
R⁴, R⁵ identiques ou différents, choisis parmi C₁-C₁₀-alkyle, C₃-C₁₀-cycloalkyle, benzyle et phényle, non substitué ou monosubstitué ou polysubstitué par C₁-C₃-alkyle,
R⁶ choisi parmi C₁-C₁₀-alkyle, C₃-C₁₀-cycloalkyle, benzyle et phényle, non substitué ou monosubstitué ou polysubstitué par C₁-C₃-alkyle.

13. Procédé selon l'une quelconque des revendications 1 à 12, **caractérisé en ce qu'**on utilise dans l'étape (a) l'eau et le mélange, contenant l'alcool de formule générale (I) et/ou l'alcool ramifié de formule générale (II), en outre le composé complexe soluble dans l'huile et l'acide de formule (III), dans un rapport volumique dans la plage de 0,5:1 à 2:1.

14. Procédé pour la préparation d'alcools ramifiés de formule générale (II)
R¹-CH₂-CH₂-CHR¹-CH₂-OH (II)
par transformation d'au moins un alcool de formule générale (I)
R¹-CH₂-CH₂-OH (I)
les groupes R¹ étant choisis parmi C₂-C₁₀-alkyle, linéaire ou ramifié, en présence d'au moins un composé complexe d'au moins un métal 8ème, 9ème ou 10ème groupe du système périodique des éléments, qui est choisi parmi les composés complexes, qui présentent au moins un ligand L¹, qui est au moins bidentate, au moins un site de coordination de L¹ étant un atome d'azote, et le composé complexe présentant une solubilité d'au moins 0,5 g/l, mesurée à température ambiante, dans au moins un des solvants toluène, m-xylène, n-hexane ou alcool de formule générale (I) ou alcool ramifié de formule générale (II), **caractérisé en ce qu'**on sépare les produits secondaires, choisis parmi les acides de formule générale (III)
R¹-CH₂-COOH (III)
sous forme d'un de leurs sels selon un procédé selon l'une quelconque des revendications 1 à 13.
